# EUROPEAN PATENT APPLICATION

(11) **EP 3 838 127 A1**
(43) Date of publication of application: **23.06.2021**
(21) Application number: 19217283.1
(22) Date of filing: 18.12.2019
(51) Int. Cl.: A61B 5/0205, A61B 5/00, G16H 40/00, A61B 5/11, A61B 5/113

(54) **DEVICE, SYSTEM AND METHOD FOR MONITORING OF A SUBJECT**

(71) Applicant: Koninklijke Philips N.V., 5656 AG Eindhoven (NL)
(72) Inventor: MATTHEIJ, Rudolphus Johannes Hubertus, 5656 AE Eindhoven (NL); ROCQUE, Mukul Julius, 5656 AE Eindhoven (NL)
(74) Representative: Philips Intellectual Property & Standards

(57) **Abstract**

The present invention relates to a device (30), system (1) and method for monitoring of a subject (100). The device comprises a processor (31) configured to obtain video data (11) of a scene including the subject, obtain audio data (21) of the scene, analyze the video data to determine one or more video-related features (13) of the subject, analyze the audio data to determine one or more audio-related features (23) of the subject, generate a video control signal (12) for controlling the acquisition and/or analysis of the video data based on the one or more audio-related features of the subject, generate an audio control signal (22) for controlling the acquisition and/or analysis of the audio data based on the one or more video-related features of the subject, and generate, based on the analysis of the audio data and the video data, monitoring information (34) indicating the status of the subject and/or an advice for taking an action.

## Description

### FIELD OF THE INVENTION

The present invention relates to a device, system and method for monitoring of a subject, such as a patient in a hospital room.

### BACKGROUND OF THE INVENTION

There exist various solutions to provide continuous and automatic patient (or, more generally, subject) monitoring. An exemplary monitoring system aims at monitoring the behavior and vital signs of patients in a general ward setting. Such a solution may rely on a camera that is able to capture a continuous stream of visual and/or depth data of one or more patients. Visual and/or depth data provide a rich source of information regarding patient behavior and their vital signs.

Certain vital signs, such as abnormalities in the patient's respiratory system can be measured, to a certain extent, by analyzing visual recordings (i.e., video data) of the patient, e.g., by evaluating body motion (e.g., chest movements caused by the respiration) or light reflections from skin region(s) of the patient to derive photoplethysmography (PPG) signals from which e.g., pulse rate and blood oxygen saturation (SpO2) can be derived by known methods.

Non-contact, remote PPG (rPPG) devices (also called camera-based device) for unobtrusive measurements have been proposed in the last decade. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

Using PPG technology, vital signs can be measured, which are revealed by minute light absorption changes in the skin caused by the pulsating blood volume, i.e., by periodic color changes of the human skin induced by the blood volume pulse. As this signal is very small and hidden in much larger variations due to illumination changes and motion, there is a general interest in improving the fundamentally low signal-to-noise ratio (SNR).

Generally, for reliably monitoring a patient it is assumed that the patient is clearly visible to the camera. Automated registration of patient behavior and vital signs by means of a camera can be challenging however: when the patient is not clearly visible to the camera, the results of analyses of visual data may be unreliable. Thus, there are a number of demanding situations, such as situations with severe motion, challenging environmental illumination conditions, or high required accuracy of the application, where an improved robustness and accuracy of the monitoring results, e.g., the determination of vital signs is required, particularly for the more critical healthcare applications.

### SUMMARY OF THE INVENTION

It is an object of the present invention to further improve the ability and reliability of monitoring a subject.

In a first aspect of the present invention a device for monitoring of a subject is presented, the device comprising a processor configured to:
- obtain video data of a scene including the subject,
- obtain audio data of the scene,
- analyze the video data to determine one or more video-related features of the subj ect,
- analyze the audio data to determine one or more audio-related features of the subj ect,
- generate a video control signal for controlling the acquisition and/or analysis of the video data based on the one or more audio-related features of the subject,
- generate an audio control signal for controlling the acquisition and/or analysis of the audio data based on the one or more video-related features of the subject, and
- generate, based on the analysis of the audio data and the video data, monitoring information indicating the status of the subject and/or an advice for taking an action.

In a further aspect of the present invention a system for monitoring of a subject is presented, the system comprising:
- a video acquisition unit configured to acquire video data of a scene including the subject,
- an audio acquisition unit configured to acquire audio data of the scene, and
- a device as disclosed herein for monitoring of the subject based on the acquired video and audio data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to make use, in addition to video data, of audio data acquired from the scene. The additional audio data may provide (additional) relevant cues about the behavior and medical status of a subject. Adding audio data to the search domain of a known monitoring device may reinforce the device's ability to increase the spectrum of vital signs that the device is able to monitor. The device, system and method according to the present invention thus allow monitoring subject behavior and vital signs by investigating audio data, such as verbal cues, acquired from the subject.

In this context, video data may include, but is not limited to, conventional RGB data and/or 3D data from a 3D (e.g., Time-of-Flight) camera or a stereo RGB camera.

Generally, by use of the evaluation of audio data and video data monitoring information indicating the status of the subject and/or an advice for taking an action are finally generated. Further, video control signals and audio control signals are generated for controlling the acquisition and/or analysis of the video data and audio data, respectively, wherein the data of the respective other type (video / audio) are preferably used to generate these control signals.

According to an embodiment the processor is configured to determine, as the one or more video-related features of the subject, one or more of the location of the subject within the scene, the orientation of the subject, the age of the subject, the gender of the subject, the sleep state of the subject, body movements of the subject, body pose of the subject, vital signs of the subject, non-verbal social signals of the subject, in particular gestures and/or facial expressions. In another embodiment the processor is configured to determine, as the one or more audio-related features of the subject, the urgency of a spoken request and/or the urgency of non-descriptive sound generated by the subject. Evaluating such features allows improving the control of the acquisition and/or analysis of the video data and audio data, respectively. For instance, if the sleep state is known and e.g., indicates that the subject is sleeping, a sudden cry of the subject, recorded as audio data, may not be interpreted as an emergency, but as sign of a dream so that no emergency call, e.g., to a nurse, is not generated.

The processor may be configured to generate a video control signal indicating one or more of a region from which video data shall be acquired, the desired resolution of video data, the zoom factor of video data, the type of body movements of the subject to be analyzed, the type of vital sign to be derived from the video data, and the type of non-verbal social signals of the subject, in particular gestures and/or facial expressions. The processor may further be configured to generate an audio control signal indicating one or more of a region from which audio data shall be acquired, a frequency range and/or amplitude range in which the audio data shall be analyzed, and a time period interval of the audio data to be acquired and/or analyzed, and a pitch of a voice of the audio data. Via such a control the acquisition and/or analysis of the data can be improved resulting in a higher reliability and/or spectrum of the final monitoring information and advice.

In another embodiment the processor is configured to generate, as monitoring information, one or more of one or more vital signs of the subject, a type of action recommended to be taken, an urgency of an action to be taken, and a notification indicating the status of the subject. The kind of monitoring information may be set by a user or may depend on the particular application of the presented monitoring solution.

In an embodiment the processor is configured to analyze the obtained audio data by differentiating between descriptive and non-descriptive sounds, and determining a separate urgency indicator for each of the descriptive sound and the non-descriptive sound, the urgency indicator indicating the urgency of an action to be taken, wherein the processor is further configured to use the determined urgency indicators for generating the monitoring information and/or the advice for taking an action. The separate processing of descriptive and non-descriptive sounds to generate separate urgency indicators increases the reliability of the generated monitoring information and advice.

Hereby, the processor may be configured to generate a common urgency indicator from the separate urgency indicators and to add the common urgency indicator to the generated monitoring information and/or the generated advice. The separate urgency indicators and/or the common urgency indicator may then be evaluated together with another urgency indicator derived from the video data, e.g., to make a decision if there is an emergency requiring to issue an emergency notification or not.

The processor may further be configured to categorize the descriptive sounds and/or the non-descriptive sounds and to assign a separate urgency indicator for the descriptive sound and/or the non-descriptive sound based on the respective category. Hereby, the categories for the descriptive sound may include if the descriptive sound represents a request of the subject and/or the categories for the non-descriptive sound include if the non-descriptive sound represent human sound indicating a healthy state or a problem or abnormality. The categories may thus further help in assigning a realistic and correct urgency indicator to the descriptive sound and/or the non-descriptive sound.

In this context, the descriptive sound may include words and specific vocalizations and the non-descriptive sound may include one or more of tone of voice, volume of voice, snoring, moaning, tachypnea, hyperpnea, air trapping, labored breathing and Cheyne-Stokes.

In another embodiment the processor is configured to generate, based on the analysis of the audio data, a preliminary advice for taking an action and to generate, based on the preliminary advice and the analysis of the video data, a final advice for taking an action. Thus, finally using both the audio data and the video data to generate a final advice reduces the number of false alarms and improves the reliability of a final advice to be correct and appropriate.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of a system and device according to the present invention,
Fig. 2 shows a schematic diagram of a first embodiment of a method according to the present invention, and
Fig. 3 shows a schematic diagram of a second embodiment of a method according to the present invention.

### DETAILED DESCRIPTION OF EMBODIMENTS

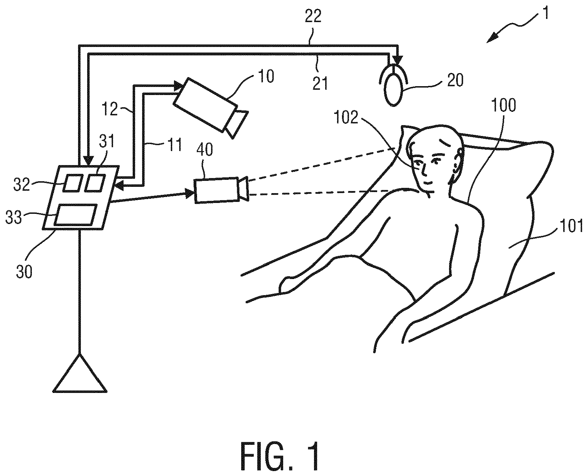

Fig. 1 shows a schematic diagram of a system 1 and device 30 according to the present invention for monitoring a subject 100. The subject 100 may be a patient, in this example a patient lying in a bed 101, e.g., in a hospital or other healthcare facility, but may also be a neonate or premature infant, e.g., lying in an incubator, or a person at home or in a different environment.

The system 1 comprises a video acquisition unit 10 configured to acquire video data 11 of a scene including the subject 100, an audio acquisition unit 20 configured to acquire audio data 21 of the scene, and the monitoring device 30 for monitoring of the subject 100 based on the acquired video and audio data 11, 21.

The video acquisition unit 10 may be configured as a camera, such as an RGB camera and/or depth camera (e.g., time-of-flight camera or stereo camera) that continuously or regularly or at controlled times records video data 11 (or image frames) of the scene, in particular of the subject 100. The recorded video data 11 are provided to the device 30 for further processing and analysis. The video acquisition unit 10 may be arranged at a wall or the ceiling of the room so that it has a direct view of the subject 100. Optionally, one or more of its parameters, such as its viewing angle, its viewing direction, its position, its recording times, its frame rate, etc. can be controlled.

The audio acquisition unit 20 may be configured as a microphone that continuously or regularly or at controlled times records audio data 21 of the scene, in particular of the subject 100. The recorded audio data 21 are provided to the device 30 for further processing and analysis. The audio acquisition unit 20 may be arranged at a wall or the ceiling of the room, preferably close to the subject 100 so that it can records any sounds generated by the subject 100. Optionally, one or more of its parameters, such as its sensitivity, its position, its recording times, its recording rate, etc. can be controlled.

While conventional 'smart speakers' require certain (combinations of) keywords (e.g., "Hey Siri!") before they will actively listen to the user, the audio signal is preferably monitored continuously so as to capture all sounds uttered by the subject, instead of only capturing purposely uttered sounds.

In an embodiment the audio acquisition unit 20 may be embodied as a smart recording device (such as a smart speaker) that is mounted near the head-part of the bed. In another embodiment it may be embodied as a smart recording device that is able to determine where the sound is originating from.

The device 30 may comprises a processor 31 or computer or any other processing means that obtains and processes the video data 11 and audio data 21 as will be explained in detail below. The device 30 may further comprise a controller 32 for generating a video control signal 12 and an audio control signal 22 for controlling the acquisition and/or analysis of the video data and the audio data, which may thus be provided to the video acquisition unit 10 and the audio acquisition unit 20. Further, the device 30 may comprise or may be connected to an interface 33 for displaying the determined information and/or for providing medical personnel with an interface to change settings of one or more components of the system 1. Such an interface 33 may comprise one or more displays, buttons, touchscreens, keyboards or other human machine interface means. Generally, the device 30 may be implemented as hard- and/or software, e.g., as appropriately programmed computer or processor.

The system 1 may optionally further comprise a light source 40 (also called illumination source), such as a lamp, for illuminating the scene, in particular the subject 100 or a particular region of interest 102 (such as the skin of the face like the cheek or forehead in case vital signs shall be derived from the video data 11 using the known method of remote PPG), with light. In particular applications, e.g., if SpO2 shall be determined from the video data, light in a predetermined wavelength range or ranges (e.g., in the red, green and/or infrared wavelength range(s)) may be used. The light reflected from said region of interest 102 in response to said illumination is detected by the camera 10. In another embodiment no dedicated light source is provided, and ambient light is used as being sufficient for illumination of the subject 100. From the reflected light only light in a number of desired wavelength ranges (e.g., green and red or infrared light, or light in a sufficiently large wavelength range covering at least two wavelength channels) may be detected and/or evaluated.

A system 1 as illustrated in Fig. 1 may, e.g., be located in a hospital, healthcare facility, elderly care facility or the like. A part from the monitoring of patients, the present invention may also be applied in other fields such as neonate monitoring, general surveillance applications, and security monitoring. The uni- or bidirectional communication between the components of the system 1 may work via a wireless or wired communication interface. Other embodiments of the present invention may include a device 30, which is not provided stand-alone, but integrated into the image acquisition unit 10.

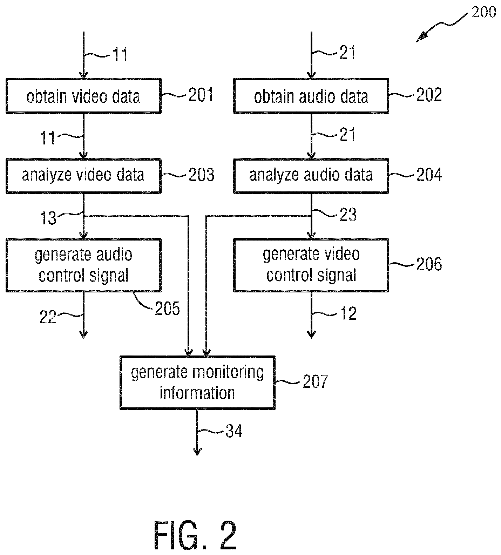

Fig. 2 shows a schematic diagram of a first embodiment of a method 200 according to the present invention, which is carried out by the device 30. Initially (steps 201, 202) video data 11 of a scene including the subject 100 (acquired by the video acquisition unit 10) and audio data 21 of the scene (acquired by the audio acquisition unit 20) are obtained (i.e., received or retrieved, preferably directly from the video acquisition unit 10 and the audio acquisition unit 20).

In subsequent steps 203, 204 the video data 11 are analyzed to determine one or more video-related features of the subject and the audio data 21 are analyzed to determine one or more audio-related features of the subject.

Video-related features 13 of the subject may include one or more of the location of the subject within the scene, the orientation of the subject, the age of the subject, the gender of the subject, the sleep state of the subject, body movements of the subject, body pose of the subject, vital signs of the subject, non-verbal social signals of the subject, in particular gestures and/or facial expressions. Conventional image processing and image recognition methods may be applied for this purpose. For instance, the above-mentioned remote PPG method or motion recognition method may be used for determining vital signs. Facial recognition or recognition of body movements may be used to determine the sleep state.

A broad range of video-related features 13 of the subject may thus be analyzed. By means of machine learning techniques, the device 30 may be able to recognize specific video-related features as specific behaviors/situations, which may form an input to control the audio recording. For instance, the device 30 may analyze the video data 11 to establish - amongst others - the location, body pose and respiratory rate of the subject in the room. Based on the detected body pose and respiratory rate of the subject (both e.g., identified by dedicated machine learning algorithms), it may be concluded that the respiratory rate of the subject may be irregular. This information may be used to control the audio acquisition unit 20 to focus its attention to a specific point in the 3D space (in particular the face region of the subject) to confirm whether the respiratory rate of the subject (as distinguishable in the audio domain) shows, indeed, irregularities.

Audio-related features 23 of the subject may include the urgency of a spoken request or spoken sounds (e.g., words, specific vocalizations, etc.) and/or the urgency of non-descriptive sound (e.g., moaning, snoring, labored breathing, respiratory abnormalities, etc.) generated by the subject. Conventional speech processing and audio processing methods may be used for this purpose.

Such urgencies may e.g. be determined by means of trained machine learning classifiers. For instance, it may be possible to recognize the spoken words itself (e.g. "I as the computer system recognize that the subject just spoke the word 'help') and compare them with items on, for example, a list/database. E.g., "help", "emergency", "problem" may be words with a high urgency indicator. The logic may be that "if a spoken word/sentence is in connection with high urgency, then assign a high urgency indicator to the meaning of the word/sentence". Further, it may be possible to recognize the severity/urgency of the vocal cue ("I as the computer system recognize that the subject sounds rather stressed/anxious"). E.g., if a person is stressed or scared, this is reflected in (the vocal characteristics of) his/her voice. Trained machine learning classifiers may be able to recognize such characteristics and assign a corresponding urgency label to it. The logic may be that "if the voice of the person contains significant characteristics of stress and/or anxiety, then assign a high urgency indicator to the context of the word/sentence".

In subsequent steps 205, 206 the video control signal 12 for controlling the acquisition and/or analysis of the video data 11 based on the one or more audio-related features 23 of the subject and the audio control signal 22 for controlling the acquisition and/or analysis of the audio data 21 based on the one or more video-related features 13 of the subject are generated. Optionally, for generating the video control signal 12 the video-related features 13 may be used in addition to the audio-related features 23, and for generating the audio control signal 22 the audio-related features 23 may be used in addition to the video-related features 13.

The video control signal 12 may indicate one or more of a region from which video data shall be acquired, the desired resolution of video data, the zoom factor of video data, the type of body movements of the subject to be analyzed, the type of vital sign to be derived from the video data, and the type of non-verbal social signals of the subject, in particular gestures and/or facial expressions. The type of social signals is related, yet different from, the type of body movements, because the perceived meaning of the body movement is at least as interesting as recognizing the type of body movement. For instance, if a subject is pointing towards a person, recognizing the type of body movement ("pointing towards something") is relevant, but it is also relevant to know where the person is point to ("point towards medical professional").

The audio control signal 22 may indicate one or more of a region from which audio data shall be acquired, a frequency range and/or amplitude range in which the audio data shall be analyzed, and a time period interval of the audio data to be acquired and/or analyzed, and a pitch of a voice of the audio data.

For instance, the audio control signal 22 may include a directed location-based analyses (e.g., "please analyze the sounds coming from the bed area; the bed area is located at distance A in direction B from your current location") and/or a directed audio-pitch analysis (e.g., "the patient is a child, please focus your monitoring efforts on the audio cues emitted in the higher-pitched audio domain"). This allows the audio acquisition unit 20 to analyze specific audio sources (and optionally to exclude audio sources that are deemed irrelevant and may therefore be considered as background noise) and come up with better informed estimations of, for example, the urgency of requests, which, in turn, increases the quality of the information generated by the device 30.

In an embodiment the processing of the audio data and the video data may be made such that the video data are evaluated first (or continuously) and that based thereon the audio control signal is generated to acquire and/or evaluate the audio data in a particular way. Hence, a steerable solution may be provided in which the evaluation of the video data steers the acquisition and/or analysis of the audio data.

In a final step 207, based on the analysis of the audio data and the video data, monitoring information 34 indicating the status of the subject and/or an advice for taking an action is generated, e.g. via a look-up table or a self-learning system. As monitoring information, one or more of one or more vital signs of the subject, a type of action recommended to be taken, an urgency of an action to be taken, and a notification indicating the status of the subject may be generated. The monitoring information may be issued via the user interface 33 or may be transmitted to another location, e.g., to a physician's computer or smartphone or to a central monitoring station for information a caregiver or monitoring staff, who may then decide what to do (e.g., to see the subject and check the situation, take a particular action (e.g., following the advice transmitted, etc.).

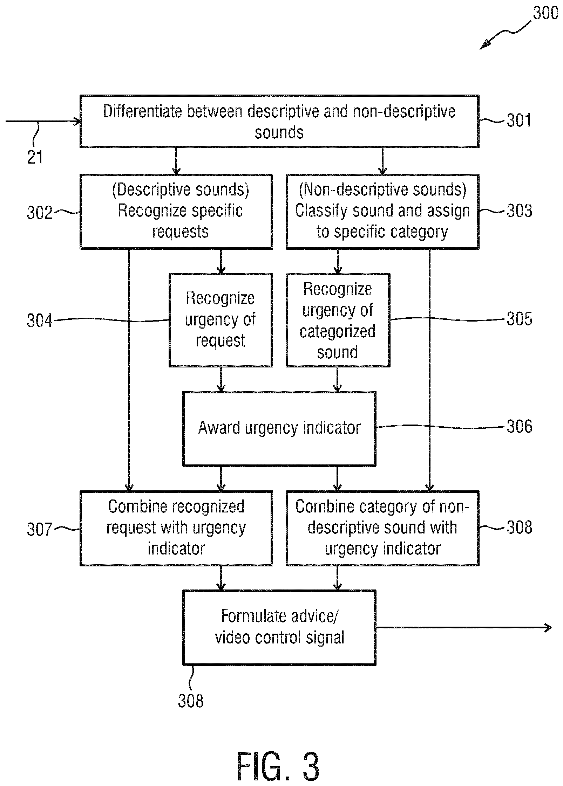

Fig. 3 shows a schematic diagram of a second embodiment of a method 300 according to the present invention that can be carried out by the device 30. This method 300 represents one possible implementation of step 204 for analyzing the audio data 21.

In a first step 301 it is differentiated between descriptive sounds and non-descriptive sounds. Advanced signal processing and machine learning techniques may be used for this and for separately analyzing both the descriptive and non-descriptive sounds in subsequent steps 302, 303. The descriptive sounds are particularly analyzed for specific requests (e.g., patient is requesting the presence of a nurse), and the non-descriptive sounds are particularly classified into specific groups (e.g., moaning, snoring, tachypnea, hyperpnea, air trapping, Cheyne-Stokes).

In subsequent step 304 for the analyzed descriptive sounds the urgency of requests is recognized and an urgency indicator is awarded to it (step 306). As an example, the request "I want to change the channel on the TV" may be recognized as less urgent than the request "My chest hurts" so that a lower urgency indicator will be assigned.

In parallel, in step 305 for the non-descriptive sounds the urgency of the non-descriptive sounds is recognized and an urgency indicator is awarded to it (step 306). As an example, snoring might receive a low urgency, moaning might receive a middle urgency, and Cheyne-Stokes breathing abnormality might receive a high urgency.

Separate urgency indicators for descriptive and non-descriptive sound are preferably obtained because audio cues may be very difficult to interpret, given that they are generally dependent on the context. For instance, a high urgency indicator may be assigned to an audio cue when it detects that an audio source shows signs of heavy breathing or a scream for help. However, the context is highly relevant, because the overall situation is positive and does not warrant a high urgency indicator. To prevent such false alarms, in the analysis of the audio data the context in which the sounds are detected is preferably taken into account. For instance, a particular situation may be recognized as a combination of "happy interaction between patient and visitor" (positive connotation) and "potential problem based on labored breathing and scream for help" (negative connotation). Thus, both findings may be taken into account and to formulate an urgency and/or advice, such as: "With the video data an eye shall be kept out for the patient; I notice some signs of anxiety, but also a lot of positive enthusiasm, so there is probably nothing going on here."

Optionally, from the separate urgency indicators a common urgency indicator may be generated in step 306, e.g. through averaging of the separate urgency indicators.

In step 307 the recognized request may be combined with the assigned urgency indicator, and in step 308 the recognized category of the non-descriptive sound may be combined with the assigned urgency indicator.

Finally, in step 309 the generated metadata (i.e., recognized request from patient or category of non-descriptive sound) and the (respective) urgency indicator(s) are used to formulate the video control signal for use by the video acquisition unit as described above. Further, optionally, audio monitoring information, e.g. an advice for use by a caregiver (for instance, notify a healthcare professional to provide further investigation) may be generated. Based on the video control signal, specific parts of the scene (e.g., the upper part of the bed/torso of the patient) may be monitored (e.g. by changing the viewing angle or zoom of the camera and/or by analyzing this part within the acquired video data). Further, it may be decided to send out a notification for assistance to a healthcare professional.

The following example shall illustrate the operation of the present invention. Initially, it is noticed that the patient utters descriptive and/or non-descriptive sounds. The device is able to differentiate between descriptive and non-descriptive sounds, recognize the meaning or category of each audio cue, and recognize the urgency of each audio cue. The findings (e.g., "DESCRIPTIVE SOUND: patient is requesting the presence of a nurse; medium urgency" or "NON-DESCRIPTIVE SOUND: patient has labored breathing; further investigation is advised; high urgency"), if necessary accompanied by a request for further investigation, is then provided to the next step of the processing.

In the next step, based on the findings (e.g., "DESCRIPTIVE SOUND: patient is requesting the presence of a nurse; medium urgency"), the device may decide to send out a notification to the designated healthcare professional. If the device receives an advice for further investigation (e.g., "NON-DESCRIPTIVE SOUND: patient has labored breathing; further investigation is advised; high urgency"), it may investigate specific parts of the scene (e.g., the upper part of the bed/torso of the patient). Based on the audio-signal guided advice, the device may decide to send out a notification to a healthcare professional.

In another example, a patient is having a nightmare while sleeping. The patient is talking in his sleep, including scared requests for help. The audio acquisition unit will recognize the non-descriptive sounds (i.e., the tone of the patient's voice), as well as the descriptive sound (i.e., the words that are uttered by the patient). The audio acquisition unit analyses both the non-descriptive as well as the descriptive sounds, and assigns the following meta-data to the sounds: <situation: patient is scared. Urgency indicator: medium> and <situation: patient calls for help. Urgency indicator: high>. Then, the audio acquisition unit formulates an advice (control) to the video acquisition unit that is overseeing the situation: "I noticed that the patient shows vocal signs of anxiety. The patient also calls for help. My indication is that the patient is in urgent need of help. I advise you to notify a nearby healthcare professional". This advice may then be used to perform a specific investigation of the situation, i.e., to assess the situation of the patient based on the video data. It is then noticed that the patient is asleep and shows visual characteristics of a person having nightmares, it is concluded that the patient is not in dire need of assistance. It may therefore be decided to take alternative actions (e.g., log the fact that the patient had a nightmare to the patient's personal file), rather than notifying a nurse. In this way, the device makes sure that no false alarms are issued to the healthcare professionals.

In still another example a patient is laying in a hospital bed. Due to his medical condition, the patient is not able to perform significant body movements. The audio acquisition unit notices that at a certain moment, the patient is laughing. The audio capturing device formulates the following meta-data: <situation: patient is happy. Urgency indicator: low>, and uses this to formulate an advice that the situation is all fine. Then, 30 seconds later, the audio acquisition unit notices that the patient makes angry sounds; the patient is cursing and grunting. The audio acquisition unit formulates the following meta-data: <situation: patient is annoyed. Urgency indicator: medium>, and uses this to advise that the patient, albeit annoyed, does not need immediate assistance. Another 30 seconds later, the audio acquisition unit notices that the patient utters happy sounds again, although this is, again, followed by a period of angry cursing and grunting. At each of these intervals, the audio acquisition unit reports the situation, varying between low and medium priority, but never recommending immediate actions. The device may, however, notice based on the video data that the patient switches often between happy and angry moods and, as such, that the patient shows erratic behavior. Hence, it may be concluded that the patient requires specific medical expertise, after which it notifies a suitable healthcare professional.

According to the present invention, audio data, in addition to video data, are used for subject monitoring, the audio data providing (additional) relevant cues about the behavior and medical status of patients. Adding audio data to the search domain of the monitoring device may reinforce the device's ability to increase the spectrum of vital signs that the device is able to monitor. This allows to monitor patient behavior and vital signs by investigating verbal cues provided by the patient.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (30) for monitoring of a subject (100), the device comprising a processor (31) configured to:
- obtain video data (11) of a scene including the subject,
- obtain audio data (21) of the scene,
- analyze the video data to determine one or more video-related features (13) of the subject,
- analyze the audio data to determine one or more audio-related features (23) of the subject,
- generate a video control signal (12) for controlling the acquisition and/or analysis of the video data based on the one or more audio-related features of the subject,
- generate an audio control signal (22) for controlling the acquisition and/or analysis of the audio data based on the one or more video-related features of the subject, and
- generate, based on the analysis of the audio data and the video data, monitoring information (34) indicating the status of the subject and/or an advice for taking an action.

2. Device as claimed in claim 1,
wherein the processor (31) is configured to determine, as the one or more video-related features of the subject, one or more of the location of the subject within the scene, the orientation of the subject, the age of the subject, the gender of the subject, the sleep state of the subject, body movements of the subject, body pose of the subject, vital signs of the subject, non-verbal social signals of the subject, in particular gestures and/or facial expressions.

3. Device as claimed in claim 1 or 2,
wherein the processor (31) is configured to determine, as the one or more audio-related features of the subject, the urgency of a spoken request and/or the urgency of non-descriptive sound generated by the subject.

4. Device as claimed in any one of the preceding claims,
wherein the processor (31) is configured to generate a video control signal indicating one or more of a region from which video data shall be acquired, the desired resolution of video data, the zoom factor of video data, the type of body movements of the subject to be analyzed, the type of vital sign to be derived from the video data, and the type of non-verbal social signals of the subject, in particular gestures and/or facial expressions.

5. Device as claimed in any one of the preceding claims,
wherein the processor (31) is configured to generate an audio control signal indicating one or more of a region from which audio data shall be acquired, a frequency range and/or amplitude range in which the audio data shall be analyzed, and a time period interval of the audio data to be acquired and/or analyzed, and a pitch of a voice of the audio data.

6. Device as claimed in any one of the preceding claims,
wherein the processor (31) is configured to generate, as monitoring information, one or more of one or more vital signs of the subject, a type of action recommended to be taken, an urgency of an action to be taken, and a notification indicating the status of the subject.

7. Device as claimed in any one of the preceding claims,
wherein the processor (31) is configured to analyze the obtained audio data by:
- differentiating between descriptive and non-descriptive sounds, and
- determine a separate urgency indicator for each of the descriptive sound and the non-descriptive sound, the urgency indicator indicating the urgency of an action to be taken, and
wherein the processor is further configured to use the determined urgency indicators for generating the monitoring information and/or the advice for taking an action.

8. Device as claimed in claim 7,
wherein the processor (31) is configured to generate a common urgency indicator from the separate urgency indicators and to add the common urgency indicator to the generated monitoring information and/or the generated advice.

9. Device as claimed in any one of claims 7 and 8,
wherein the processor (31) is configured to categorize the descriptive sounds and/or the non-descriptive sounds and to assign a separate urgency indicator for the descriptive sound and/or the non-descriptive sound based on the respective category.

10. Device as claimed in claim 9,
wherein the categories for the descriptive sound include if the descriptive sound represents a request of the subject and/or the categories for the non-descriptive sound include if the non-descriptive sound represent human sound indicating a healthy state or a problem or abnormality.

11. Device as claimed in any one of claims 7 to 10,
wherein the descriptive sound includes words and specific vocalizations and the non-descriptive sound includes one or more of tone of voice, volume of voice, snoring, moaning, tachypnea, hyperpnea, air trapping, labored breathing and Cheyne-Stokes.

12. Device as claimed in any one of the preceding claims,
wherein the processor (31) is configured to generate, based on the analysis of the audio data, a preliminary advice for taking an action and to generate, based on the preliminary advice and the analysis of the video data, a final advice for taking an action.

13. System (1) for monitoring of a subject (100), the system comprising:
- a video acquisition unit (10) configured to acquire video data of a scene including the subject,
- an audio acquisition unit (20) configured to acquire audio data of the scene, and
- a device (30) as claimed in any one of the preceding claims for monitoring of the subject based on the acquired video and audio data.

14. Method for monitoring of a subject (100), the method comprising:
- obtaining video data (11) of a scene including the subject,
- obtaining audio data (21) of the scene,
- analyzing the video data to determine one or more video-related features (13) of the subject,
- analyzing the audio data to determine one or more audio-related features (23) of the subject,
- generating a video control signal (12) for controlling the acquisition and/or analysis of the video data based on the one or more audio-related features of the subject, and
- generating an audio control signal (22) for controlling the acquisition and/or analysis of the audio data based on the one or more video-related features of the subject, and
- generating, based on the analysis of the audio data and the video data, monitoring information (34) indicating the status of the subject and/or an advice for taking an action.

15. Computer program comprising program code means for causing a computer to carry out the steps of the method as claimed in claim 14 when said computer program is carried out on the computer.
